# EUROPEAN PATENT SPECIFICATION

(11) **EP 4 321 118 B1**
(45) Date of publication and mention of the grant of the patent: **17.09.2025**
(21) Application number: 23191087.8
(22) Date of filing: 11.08.2023
(51) Int. Cl.: A61B 17/80, A61B 17/16

(54) **BONE PLATE WITH TAMP WINDOW FOR TIBIA PLATEAU FRACTURE**
KNOCHENPLATTE MIT EINEM STOPFFENSTER FÜR TIBIAPLATEAUBRUCH
PLAQUE OSSEUSE AVEC FENÊTRE DE TAMPON POUR FRACTURE DE PLATEAU TIBIAL

(30) Priority: 12.08.2022 US 202263397488 P
(43) Date of publication of application: 14.02.2024
(73) Proprietor: Stryker European Operations Limited, Carrigtwohill, Co. Cork T45 HX08 (IE)
(72) Inventor: TARKIN, Ivan, Sewickley, 15143 (US)
(74) Representative: Maiwald GmbH

(56) References cited:
- US-A1- 2011 218 576
- US-A1- 2012 053 586
- US-A1- 2016 278 824
- US-A1- 2022 240 991

## Description

### BACKGROUND OF THE INVENTION

When a bone is damaged or fractured, bone plates are commonly attached to the outside surface of the damaged bone to stabilize the area and promote healing of the bone. Screws (locking and nonlocking) are applied through the plates on either side of the broken bone and the plate is left in place at least until healing has occurred (*i.e.,* fracture union). Generally, the plates have head and shaft portions, a bone contacting side and a side facing away from the bone with a plurality of holes extending through the two surfaces and thickness of the plate. The plates may be fixed to the bone with different types of fixation elements, such as locking and non-locking screws. The plates may also be utilized in connection with bone growth promoting materials to facilitate better and stronger healing of the bone.

Bone plates are typically employed for periarticular fractures - fractures around or involving joints. These periarticular plates are often designed for specific anatomic regions. Such plates are known for example from US 2012/053586 A1, US 2022/240991 A1, US 2016/278824 A1 and US 2011/218576 A1. However, knee fractures involving the top portion of the tibia (the tibia plateau) break in certain reliable patterns (commonly referred to as Schatzker fractures). Current designs have not been specifically engineered to facilitate care of the so called Schatzker II variant (involving a lateral split depression). This requires the use of plates designed for other Schatzker fractures (*e.g*., Schatzker VI fractures), which may result in an ineffective treatment method. Indeed, optimal plates designed for use in fixing Schatzker II fractures should be more flexible and should accommodate the need to elevate the depressed joint surface while maintaining the width of the tibia plateau.

Thus, there exists a need for specialized plates for treating different fractures, such as Schatzker II fractures.

### BRIEF SUMMARY OF THE INVENTION

The present invention is defined in claim 1 while preferred embodiments are set forth in the dependent claims.

Associated surgical methods are also described herein to aid understanding the invention. These methods do not form part of the claimed invention.

This invention generally relates to Schatzker II bone plate systems for the internal fixation of tibial plateau fractures, and to the insertion and placement of bone graft with a tamp through a window formed through a bone plate. The particular plate shown and described herein is a plate designed for internal fixation of a fracture around the knee joint with depressed joint cartilage. A window is engineered in the plate to accommodate a bone hole used to insert a tamp to correct joint surface irregularity as well as bone grafting of the inevitable bone defect.

The presently disclosed bone plating system allows for a tamp to be inserted into a window on the surface of the bone plate. The window is sized and shaped to receive a portion of the tamp therethrough. The window also allows the plate to be more easily bendable and/or flexible.

According to the present invention, a tibia Schatzker II fracture bone plate system comprises a bone plate including an elongated shaft portion and a head portion connected to and monolithic with the elongated shaft portion, the shaft and head portions in angled relationship from approximately 45 degrees to 60 degrees between shaft and head portion, including a bone contacting bottom surface and an opposite top surface; a hole extending from the top surface to the bone contacting bottom surface; and a window extending from the top surface to the bone contacting bottom surface and being adapted to permit the head portion to be bent over the shaft; and a tamp including a head, wherein the head is sized and shaped to pass through the window.

In accordance with other embodiments, the window can define an area that is used as an access point for a head of a tamp. The perimeter of the window can be at least twice as large as the perimeter of the hole. Still further, the tamp can be placed through the window at an angle within the range of 30-60 degrees with respect to the elongated shaft portion. The bone plate may define a length generally in a proximal-distal direction and a width along a direction orthogonal to the length, e.g., between side edges of the bone plate. Furthermore, the window may also define a length extending along the length of the bone plate generally in the proximal-distal direction and a width in a direction orthogonal to the length. The width of the window may change along the length of the window. The window may include a window edge defining a perimeter of the window. The window edge may include a proximal edge extending along the width of the window, a distal edge opposing the proximal edge, and opposing side edges connecting the proximal and distal edges. The width of the window along the proximal edge may be greater than the width of the window along the distal edge. The width of the window may taper as the window extends distally along the length. At least a portion of the window edge may extend generally parallel to a portion of an edge of the bone plate. One or both of the side edges of the window may extend parallel to corresponding side edges of the bone plate (*e.g.,* the bone plate side edges nearest to each of the window side edges). The window may define a window edge comprising straight portions connected by rounded portions. The straight portions of the window edge may comprise the proximal edge, distal edge and side edges, and each adjacent edge may be connected by a rounded portion of the window edge. A first pair of opposing straight portions among the straight portions of the window edge may extend parallel to one another. The proximal edge and the distal edge of the window edge may be the pair of opposing straight portions extending parallel to one another. A second pair of opposing straight portions among the straight portions of the window edge may extend oblique to one another. The pair of opposing side edges may be the straight portions of the window edge which extend oblique to one another. The hole may be located distal to the window along the bone plate. The window may be located on the head portion of the bone plate. The hole may be located on the head portion of the bone plate. The hole and the window may be located on a distal portion of the head portion. The hole may be located adjacent the window on the head portion, and the hole and the window may be located between a first set of screw holes on the head portion and a second set of screw holes on the elongated shaft portion.

A bone plate system according to embodiments of the present invention comprises an opening including a length in a first direction running along the elongated shaft portion and a width in a second direction that is orthogonal to the first direction wherein the length is greater than the width, and the opening extends from the upper surface to the bone contacting bottom surface. The opening is positioned between the head portion and the shaft portion. A window extends from the upper surface to the bone contacting bottom surface and has an area larger than an area of the hole. The tamp includes a head having an area smaller than the area of the window.

In accordance with other embodiments, the opening may have an oblong shape and may be located below (*e.g*., distal to) the window. The perimeter of the window may be at least twice as large as the perimeter of the opening. The tamp may be placed through the window at an angle within the range of 30-60 degrees with respect to the elongated shaft portion. The opening may have a perimeter defined by two opposing straight portions and two opposing rounded portions connecting the straight portions. The opening may be oblong in the direction of the length of the bone plate. A length of the opening may be greater than a width of the opening.

Also described herein, but not claimed, is a method of repairing a fracture of a bone. The method includes the steps of placing a bone-contacting surface of a bone plate adjacent to the bone, inserting a tamp through a window extending through the bone plate, inserting a bone graft material through the window and impacting the bone graft material using the tamp.

In accordance with other examples of this method, the tamp may be used to correct an articular surface located on a tibial plateau. The step of inserting the tamp through the window may occur at an angle within the range of 30-60 degrees with respect to a portion of the bone plate.

Also described herein, but not claimed, is a method of repairing a split depression lateral tibia plateau fracture. The method includes the steps of reducing the split in the tibia, attaching a plate to the tibia, creating a hole in the tibia through a window in the plate, inserting a tamp through the window to repair an irregular articular surface of the tibia and inserting graft material or other bone void filler through the window to fill a void in the tibia. In certain examples, the step of inserting the tamp through the window occurs at an angle within the range of 30-60 degrees with respect to a portion of the bone plate.

### BRIEF DESCRIPTION OF THE DRAWINGS

FIG. 1 is a top view of a bone plate according to one embodiment of the invention.
FIG. 2 is a side view of the bone plate of FIG. 1.
FIG. 3 is an enlarged view of the proximal head portion of the bone plate of FIG. 1.
FIG. 4 shows the bone plate of FIG. 1 located on the proximal tibia.
FIG. 5 is an enlarged view of the window distal head portion on the bone plate.
FIGS. 6a-d depict example steps of using the bone plate of FIG. 1.
FIG. 7 shows the bone plate of FIG. 1 with a tamp.
FIG. 8 is an enlarged view of the head of the tamp shown in FIG. 7.
FIG. 9 is a plan view of a screw for use in connection with the present invention.

### DETAILED DESCRIPTION OF THE PREFERRED EMBODIMENTS

As used herein unless stated otherwise, the term "anterior" means toward the front part of the body, and the term "posterior" means toward the back part of the body. When referring to specific directions in the following discussion of a certain device, the terms "proximal" and "distal" are to be understood in regard to the device's orientation and position during exemplary application to human body. Thus, the term "proximal" means closer to the operator or in a direction toward the operator, and the term "distal" means more distant from the operator or in a direction away from the operator. In addition, the terms "about," "generally," and "substantially" are intended to mean that deviations from absolute are included within the scope of the term so modified.

Turning now to the figures, a bone plate 10 is shown and will be discussed below. The particular design shown is intended for the internal fixation of a fractured proximal tibia of a patient, and in particular, for use in treating a Schatzker II fracture. However, it is contemplated that plates in accordance with the present invention could be tailored for use in connection with the fixing of other types of fractures. The invention is not so limited to just the specific design and use disclosed herein.

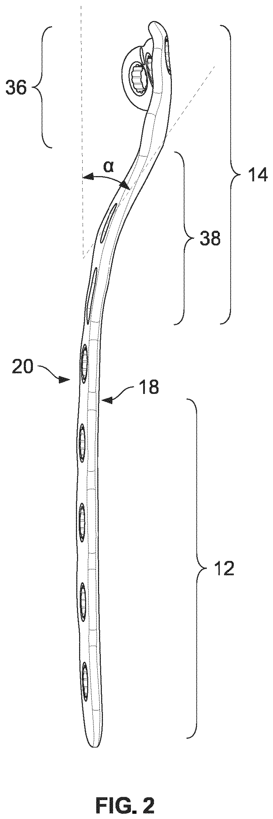

Plate 10 includes a shaft portion 12 that is formed monolithically with a head portion 14, a bone contacting bottom surface 20 (*see* FIG. 2), and an opposite top surface 18. Both shaft portion 12 and head portion 14 include a plurality of holes 16 extending between top and bottom surfaces through a thickness of plate 10. The plurality of holes 16 are capable of receiving both locking and non-locking screws (not shown). The particular hole design shown is substantially similar to certain of those disclosed in U.S. Patent No. 11,039,865, but it is contemplated that the holes can be of any design known in the art. This includes holes designed to receive locking, non-locking or both types of screws. It is also contemplated that the holes could be designed to receive other types of fixation devices, such as pegs or the like.

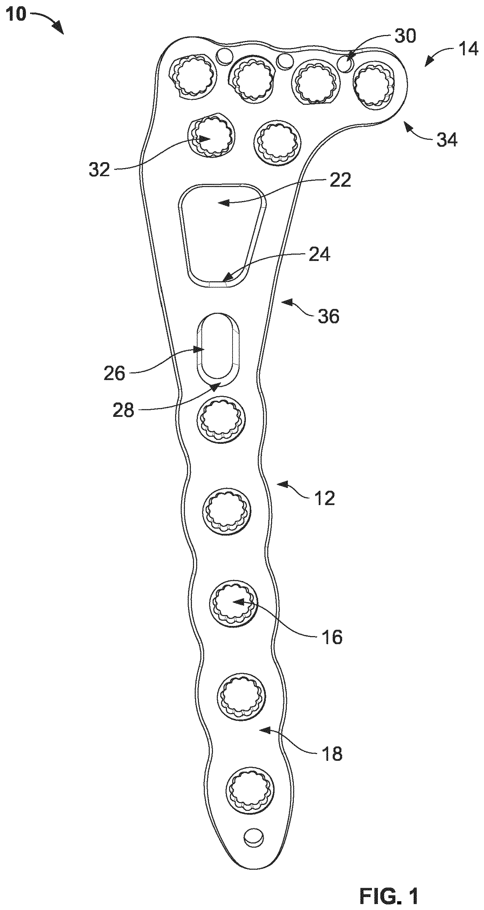

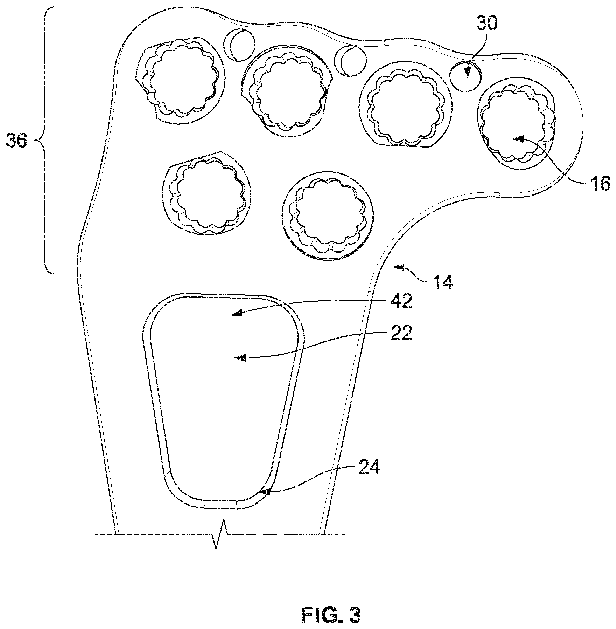

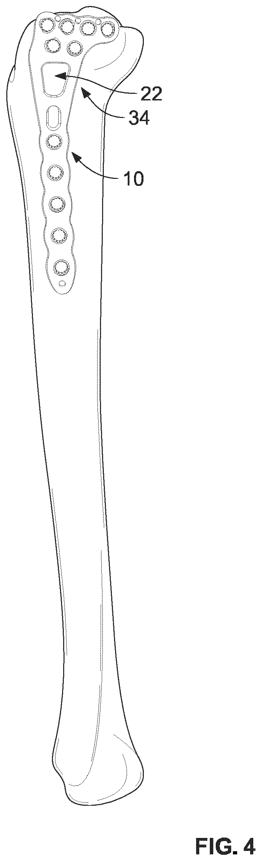

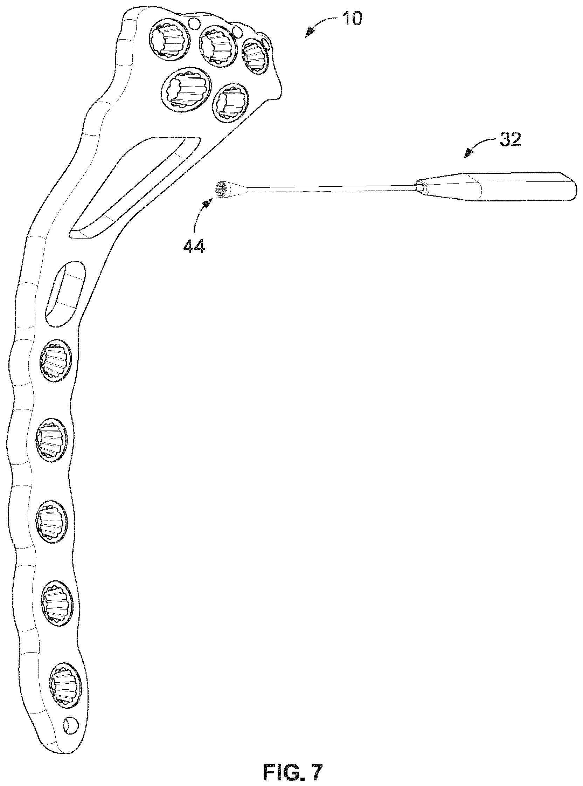

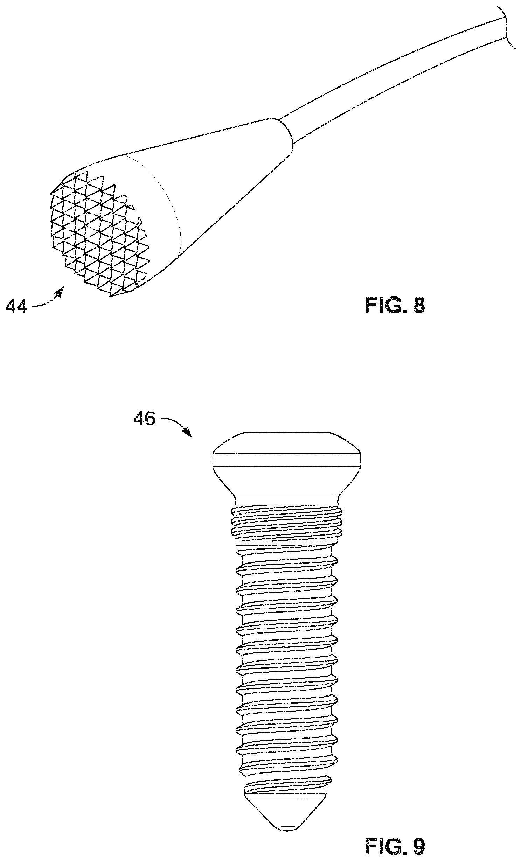

Referring to FIG. 1 and FIG. 3, the head portion 14 defines a window 22 that is sized for the reception of a portion of a tamp. This tamp can be utilized to reduce a bone fracture, as well as for applying a bone graft material near the tibia plateau region (or metaphysis) 34 (*see* FIG. 4). Window 22, like holes 16 and 30, extends between top and bottom surfaces 18, 20 through a thickness of plate 10 and includes a window edge 24 defining the size and shape of the window. In the embodiment shown, edge 24 is sized and shaped to encompass a portion of a tamp, such as tamp head 44 of tamp 32 shown in FIGS. 7 and 8. The window is positioned such that tamp can be placed therethrough at an angle of approximately 45 degrees (±15 degrees) with respect to the shaft of the plate and/or the shaft of the tibia. This placement angle permits the proper reduction of the fracture, as well as the proper placement of bone graft.

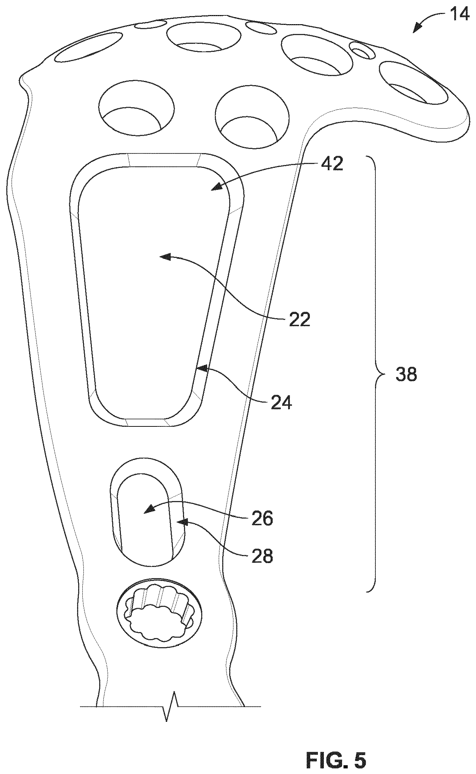

Additionally, with reference to FIGS. 2, 3, and 5, head portion 14 includes k-wire holes 30 and an oblong hole 26 located near or at the connection with shaft portion 12. More particularly, a proximal portion 36 (best shown in FIG. 3) of head portion 14 extends laterally and includes screw holes 16 and k-wire holes 30, while distal portion 38 (best shown in FIG. 5) of head portion 14 encompasses window 22 and oblong hole 28. The width of the bone plate in distal portion 38, decreases when the bone plate is viewed in a direction from a proximal end to a distal end of plate 10. The specific sizes and shapes shown in the drawings are designed with Schatzker II fractures in mind but can vary depending upon the particular application for plate 10. This includes varying the sizes and shapes of window 22 to accommodate different sized and shaped tamps.

As shown in Fig. 5, oblong hole 26 includes an inner surface 28 separating bottom surface 20 and top surface 18. This surface can facilitate the reduction of different fracture parts through placement of a lag screw or the like. In other words, surface 28 may force the movement of such a screw in a given direction upon placement therethrough and into the bone. This is a well-known type of feature of bone plates and any known design can be employed in plate 10.

Plate 10 exhibits an angled relationship of approximately 45 degrees (±15 degrees) between shaft 12 and head 14. This facilitates the aforementioned tamp window angle. In the particular embodiment shown, head 14 is angled in multiple directions with respect to shaft 12. Moreover, the inclusion of window 22 permits the head 14 to be bent more easily than typical bone plates, e.g., bone plates not including a window and having more material across a width of the plate. This is particularly useful in treating the different anatomies of different patients. Again, while shown for the particular uses discussed herein, plate 10 and its features can be utilized in other types of fracture fixation procedures, and the flexibility of the plate can be useful in such applications as well.

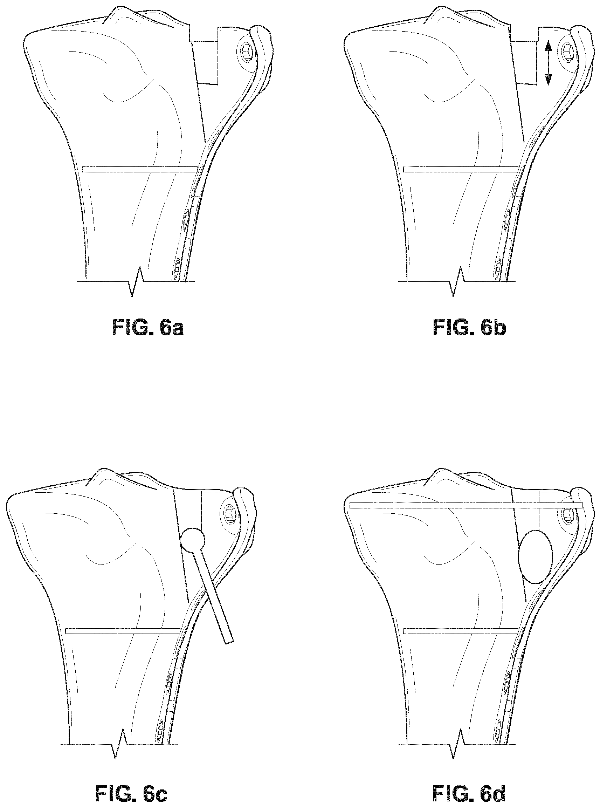

Turning now to FIGS. 6a-d, one use of plate 10 will now be discussed. In FIG. 6a plate 10 is temporarily fixed to bone surface 34 via the use of a screw or other fixation device. While a line representing a screw is shown placed through shaft 12, FIG. 9 depicts a screw 46 that can be utilized in accordance with the present invention. This step can include using one or more such fixation devices placed in one or both of shaft 12 and/or head 14. Plate 10 can then be used as a reduction aid via the buttressing function of the plate. For instance, as shown in FIG. 6b, movement of the plate can begin to reduce fracture 40. Again, plate 10 can be easily bent to allow for more or less reduction leverage, as well as to best cooperate with the bone anatomy. Tamp 32 (represented by an arrowed line in FIG. 6c) is then inserted through window 22 to further reduce fracture 40 and ultimately correct the articular surface of the bone 34. Plate 10 is then proximally fixed to the bone surface 34, for instance, by inserting screw 46 through head 14. At the same time, bone graft material 42 can be inserted through window 22 to fill any leftover bone voids. This can include using tamp 32 to push the graft material into place.

Essentially, repair of a split depression lateral plateau fracture in accordance with the present invention would include the steps of reducing the split and buttressing with plate 10. Subsequently the irregular articular surface would be addressed by creating a metaphyseal hole in the region of plate window 22. Tamp 32 would then be inserted to precisely align the impacted joint surface. The inevitable void created by fracture approximation could then be filled with autograft, allograft, or other bone void filler, by again using tamp 32 or some other instrument (other instruments are not claimed).

FIGS. 7 and 8 show tamp 32 in more detail. While the embodiment shown is a prior art device, it is contemplated that tamps specifically designed for cooperating with specially sized and shaped windows 22 are contemplated hereunder. It is also contemplated to utilize an impact device, such as a hammer, with tamp 32 to facilitate any of the foregoing steps. Moreover, it is to be understood that tools (not claimed) other than tamp 32 could be utilized with plate 10. Indeed, window 22 can be sized and shaped to cooperate with any tool useful in a given procedure. While the head of tamp 32 is shown in more detail in FIG. 8, it is contemplated to utilize tamps having different head features. For instance, the head could be round or oval and the surface of the head could be jagged or smooth. Of course, window 22 could be modified from that shown in the drawings to cooperate with these different tamp designs.

Plate 10 can be constructed of various materials, including surgical metals such as titanium and titanium alloy. It may be preferable to form plate 10 of a material that both facilitates the aforementioned bending of portions of the plate, as well as to permit fixation devices placed through holes 16 to become affixed to the plate. It is also contemplated to provide plates in accordance with the present invention in a kit with multiple sizes that may be selected based upon the patient anatomy and/or type of fracture being fixated. Furthermore, plates in accordance with the present invention could be designed to be specific to a given patient's anatomy. These are typically referred to as patient-specific plates and can be generated utilizing preoperative planning techniques.

## Claims

1. A tibia Schatzker II fracture bone plate system comprising:
a bone plate (10) including:
an elongated shaft portion (12) and a head portion (14) connected to and monolithic with the elongated shaft portion (12), the shaft and head portions (12, 14) in angled relationship from approximately 45 degrees to 60 degrees between shaft (12) and head portion (14),
including a bone contacting bottom surface (20) and an opposite top surface (18);
a hole (26) extending from the top surface (18) to the bone contacting bottom surface (20); and
a window (22) extending from the top surface (18) to the bone contacting bottom surface (20) and being adapted to permit the head portion (14) to be bent over the shaft (12); and
a tamp (32) including a head (44), wherein the head (44) is sized and shaped to pass through the window (22).

2. The system of claim 1 wherein the window (22) defines an area.

3. The system of claim 2, wherein the area is configured to be used as an access point for the head (44) of the tamp (32).

4. The system of any one of claims 1-3, wherein a perimeter of the window (22) is at least twice as large as a perimeter of the hole (26).

5. The system of any one of claims 1-4, wherein the tamp (32) is configured to be placed through the window (22) at an angle within the range of 30-60 degrees with respect to the elongated shaft portion (12).

6. The system of any one of claims 1-5, wherein the window (22) defines a length in a first proximal-distal direction and the window (22) defines a width in a second direction orthogonal to the first direction, and wherein the width of the window (22) changes along the length.

7. The system of claim 6, wherein the width of the window (22) along a proximal edge (24) of the window (22) is greater than the width of the window along a distal edge (24) of the window (22).

8. The system of any one of claims 6-7, wherein the width of the window (22) tapers as the window (22) extends distally.

9. The system of any one of claims 1-8, wherein at least a portion of a window edge (24) extends parallel to a portion of an edge of the bone plate (10).

10. The system of any one of claims 1-9, wherein the window (22) defines a window edge (24) comprising straight portions connected by rounded portions.

11. The system of claim 10, wherein a first pair of opposing straight portions among the straight portions of the window edge (24) extend parallel to one another.

12. The system of claim 11, wherein a second pair of opposing straight portions among the straight portions of the window edge (24) extend oblique to one another.

13. The system of any one of claims 1-12, wherein the hole (26) is located distal to the window (22) along the bone plate (10).

14. The system of any one of claims 1-13, wherein the window (22) and the hole (26) are located on the head portion (14) of the bone plate (10).

15. The system of any one of claims 1-14, wherein the hole (26) is located adjacent the window (22) on the head portion (14), the hole (26) and the window (22) located between a first set of screw holes (16) on the head portion (14) and a second set of screw holes (16) on the elongated shaft portion (12).

## Patentansprüche

1. Tibia-Schatzker-II-Fraktur-Knochenplattensystem, umfassend:
eine Knochenplatte (10) mit:
einen länglichen Schaftabschnitt (12) und einen Kopfabschnitt (14), der mit dem länglichen Schaftabschnitt (12) verbunden und monolithisch mit diesem ausgebildet ist, wobei der Schaft- und der Kopfabschnitt (12, 14) in einem Winkel von etwa 45 Grad bis 60 Grad zwischen dem Schaftabschnitt (12) und dem Kopfabschnitt (14) angeordnet sind und
eine knochenberührende untere Fläche (20) und eine gegenüberliegende obere Fläche (18) aufweisen;
ein Loch (26), das sich von der oberen Fläche (18) zu der knochenberührenden unteren Fläche (20) erstreckt; und
ein Fenster (22), das sich von der oberen Fläche (18) zu der knochenberührenden unteren Fläche (20) erstreckt und so beschaffen ist, dass es dem Kopfabschnitt (14) ermöglicht ist, über den Schaft (12) gebogen zu sein; und
einen Stempel (32) mit einem Kopf (44), wobei der Kopf (44) so bemessen und geformt ist, dass er durch das Fenster (22) passt.

2. System nach Anspruch 1, wobei das Fenster (22) einen Bereich definiert.

3. System nach Anspruch 2, wobei der Bereich so konfiguriert ist, dass er als Zugangspunkt für den Kopf (44) des Stempels (32) verwendet werden kann.

4. System nach einem der Ansprüche 1 bis 3, wobei ein Umfang des Fensters (22) mindestens doppelt so groß ist wie ein Umfang des Lochs (26).

5. System nach einem der Ansprüche 1 bis 4, wobei der Stempel (32) dazu konfiguriert ist, durch das Fenster (22) in einem Winkel im Bereich von 30 bis 60 Grad in Bezug auf den länglichen Schaftabschnitt (12) platziert zu werden.

6. System nach einem der Ansprüche 1-5, wobei das Fenster (22) eine Länge in einer ersten proximal-distalen Richtung definiert und das Fenster (22) eine Breite in einer zweiten Richtung orthogonal zu der ersten Richtung definiert, und wobei sich die Breite des Fensters (22) entlang der Länge ändert.

7. System nach Anspruch 6, wobei die Breite des Fensters (22) entlang eines proximalen Rands (24) des Fensters (22) größer ist als die Breite des Fensters entlang eines distalen Rands (24) des Fensters (22).

8. Das System nach einem der Ansprüche 6-7, wobei sich die Breite des Fensters (22) bei einer distalen Erstreckung des Fensters (22) verjüngt.

9. System nach einem der Ansprüche 1-8, wobei mindestens ein Abschnitt eines Fensterrands (24) parallel zu einem Abschnitt eines Rands der Knochenplatte (10) verläuft.

10. System nach einem der Ansprüche 1-9, wobei das Fenster (22) einen Fensterrand (24) definiert, der gerade Abschnitte umfasst, die durch abgerundete Abschnitte verbunden sind.

11. System nach Anspruch 10, wobei sich ein erstes Paar gegenüberliegender gerader Abschnitte der geraden Abschnitte des Fensterrands (24) parallel zueinander erstrecken.

12. System nach Anspruch 11, wobei sich ein zweites Paar gegenüberliegender gerader Abschnitte der geraden Abschnitte des Fensterrands (24) schräg zueinander erstrecken.

13. System nach einem der Ansprüche 1-12, wobei das Loch (26) distal zum Fenster (22) entlang der Knochenplatte (10) angeordnet ist.

14. System nach einem der Ansprüche 1-13, wobei das Fenster (22) und das Loch (26) am Kopfabschnitt (14) der Knochenplatte (10) angeordnet sind.

15. System nach einem der Ansprüche 1-14, wobei das Loch (26) angrenzend zu dem Fenster (22) auf dem Kopfabschnitt (14) angeordnet ist, wobei das Loch (26) und das Fenster (22) zwischen einem ersten Satz von Schraublöchern (16) auf dem Kopfabschnitt (14) und einem zweiten Satz von Schraublöchern (16) auf dem länglichen Schaftabschnitt (12) angeordnet sind.

## Revendications

1. Système de plaque osseuse pour fracture de type Schatzker II du plateau tibial comprenant :
une plaque vissée (10) comportant :
une partie tige allongée (12) et une partie tête (14) reliée à la partie tige allongée (12) et monolithique avec cette dernière, les parties tige et tête (12, 14) en relation d'angle allant approximativement de 45 degrés à 60 degrés entre la partie tige (12) et la partie tête (14),
comportant une face inférieure (20) en contact avec l'os et une face supérieure (18) disposée à l'opposé ;
un trou (26) s'étendant de la face supérieure (18) à la face inférieure (20) en contact avec l'os ; et
une fenêtre (22) s'étendant de la face supérieure (18) à la face inférieure (20) en contact avec l'os et étant adaptée pour permettre à la partie tête (14) d'être courbée au-dessus de la tige (12) ; et
un tampon (32) comportant une tête (44), ladite tête (44) étant dimensionnée et formée de manière à pouvoir passer à travers la fenêtre (22).

2. Système selon la revendication 1, dans lequel la fenêtre (22) définit une zone.

3. Système selon la revendication 2, dans lequel la zone est configurée pour être utilisée comme un point d'accès pour la tête (44) du tampon (32).

4. Système selon l'une quelconque des revendications 1 à 3, dans lequel un périmètre de la fenêtre (22) est au moins deux fois plus large qu'un périmètre du trou (26).

5. Système selon l'une quelconque des revendications 1 à 4, dans lequel le tampon (32) est configuré de manière à être placé à travers la fenêtre (22) à un angle situé dans une plage allant de 30 à 60 degrés par rapport à la partie tige allongée (12).

6. Système selon l'une quelconque des revendications 1 à 5, dans lequel la fenêtre (22) définit une longueur dans une première direction proximale-distale et la fenêtre (22) définit une largeur dans une deuxième direction orthogonale par rapport à la première direction, et dans lequel la largeur de la fenêtre (22) change le long de la longueur.

7. Système selon la revendication 6, dans lequel la largeur de la fenêtre (22) le long d'un bord proximal (24) de la fenêtre (22) est supérieure à la largeur de la fenêtre le long d'un bord distal (24) de la fenêtre (22).

8. Système selon l'une quelconque des revendications 6 à 7, dans lequel la largeur de la fenêtre (22) se rétrécit au fur et à mesure que la fenêtre (22) s'étend dans le sens distal.

9. Système selon l'une quelconque des revendications 1 à 8, dans lequel au moins une partie d'un bord de fenêtre (24) s'étend parallèlement à une partie d'un bord de la plaque osseuse (10).

10. Système selon l'une quelconque des revendications 1 à 9, dans lequel la fenêtre (22) définit un bord de fenêtre (24) comprenant des parties droites reliées par des parties arrondies.

11. Système selon la revendication 10, dans lequel une première paire de parties droites opposées parmi les parties droites du bord de fenêtre (24) s'étend parallèlement l'une par rapport à l'autre.

12. Système selon la revendication 11, dans lequel une deuxième paire de parties droites opposées parmi les parties droites du bord de fenêtre (24) s'étend obliquement l'une par rapport à l'autre.

13. Système selon l'une quelconque des revendications 1 à 12, dans lequel le trou (26) est situé de manière distale par rapport à la fenêtre (22) le long de la plaque osseuse (10).

14. système selon l'une quelconque des revendications 1 à 13, dans lequel le fenêtre (22) et le trou (26) se situent sur la partie tête (14) de la plaque osseuse (10).

15. Système selon l'une quelconque des revendications 1 à 14, dans lequel le trou (26) est situé adjacent à la fenêtre (22) sur la partie tête (14), le trou (26) et la fenêtre (22) situés entre un premier ensemble de trous de vis (16) sur la partie tête (14) et un deuxième ensemble de trous de vis (16) sur la partie tige allongée (12).
